# EUROPEAN PATENT APPLICATION

(11) **EP 3 295 932 A2**
(43) Date of publication of application: **21.03.2018**
(21) Application number: 17020376.4
(22) Date of filing: 23.08.2017
(51) Int. Cl.: A61K 9/00, A61K 31/4985, A61K 47/69

(54) **STABLE ODF COMPOSITION CONTAINING HARDLY SOLUBLE THERAPEUTIC AGENT**

(30) Priority: 15.09.2016 CZ 20160570
(71) Applicant: Zentiva K.S., 102 37 Praha 10 (CZ)
(72) Inventor: Stasiak, Pawel, 80-180 Gdansk (PL); Kluk, Anna, 10-691 Olsztyn (PL); Hanzlik, Pavel, 190 00 Praha 9 (CZ); Sedlak, Pavel, 149 00 Praha 4 (CZ); Kral, Vladimir, 120 00 Praha 2 (CZ); Mikes, Petr, 463 31 Mnisek u Liberce (CZ); Saman, Ales, 463 34 Hradek nad Nisou (CZ)
(74) Representative: Jirotkova, Ivana

(57) **Abstract**

The present subject relates to the composition of the solution used for electrospinning process in order to prepare orodispersible films as well as the composition of ODFs, comprising hardly soluble therapeutic agents. The subject further relates to method of preparation of the stable solution, stabilization procedures and effective taste masking method.

## Description

### Field of the Invention

The present invention relates to the composition of the solution used for electrospinning process in order to prepare orodispersible films as well as the composition of ODFs, comprising hardly soluble therapeutic agents. The invention further relates to method of preparation of the stable solution, stabilization procedures and effective taste masking method.

### Background Art

Orodispersible films (ODFs) have recently gained much attention, as it is a very beneficial formulation for pediatric and geriatric patients, where the difficulty of swallowing of the standard solid drug forms needs to be eliminated. Orodispersible films either disintegrate quickly in the oral cavity and can be swallowed easily or stay in the particular place in the mouth due to their mucoadhesive properties. Thus ODFs are used as rapid release products and as buccoadhesive or swallowed controlled drug release systems.

Generally, oral strips are manufactured by casting method - preparation of solution or suspension, which is left for solvent evaporation. In this method melt polymer can also be used. Unfortunately this production method is considered as problematic, since it is difficult to achieve homogenous assay and thickness of the films, especially in a bigger scale. Other techniques used for ODFs manufacturing are hot melt extrusion and ink jet printing on placebo films (J. Control Release 206 (2015) 1-19). The newest method of ODF production is electrospinning - a process, where polymer nanofibers (diameter from few nanometers to several micrometers) can be produced using an electrostatically driven jet of polymer solution or polymer melt. The advantages of electrospinning method for ODFs production have been described in detail in a Zentiva's previous patent (EP2813212A). Due to the porous structure of polymeric nanofibers, obtained ODFs, in comparison with products obtained by casting method, demonstrate favorable properties, including homogeneity of thickness and assay, faster disintegration and immediate dissolution.

The use of electrostatic forces to form ultrafine fibers was first patented by Formhals in 1934 (US1975504). Since then, this technique had been known as electrospinning and had gained much interest on different applications, including filtration (J. Membr. Sci. 281 (2006) 581-586, J. Membr. Sci. 315 (2008) 11-19, J. Adv. Mater. 34 (2002) 44-55), cosmetic mask (CN101390814-A, US2009031691-A1), military protective clothing (US patent KR2010048661-A, J. Appl. Polym. Sci. 102 (2006) 3430-3437, J. Appl. Polym. Sci. 125 (2012) 4135-4141), nano-sensor (IEEE Sens. J. 8 (2008) 951-953, Biomacromolecules 9 (2008) 2087-2090), energy-related applications (J. Power Sources 196 (2011) 4886-4904), wound dressings (Polym. Adv. Technol. 21 (2010) 77-95, J. Biomed. Mater. Res. B. 67B (2003) 675-679, J. Mater. Chem. B. 1 (2013) 4531-4541), drug delivery (J. Nanomater. (2013) 1-22, Biomaterials 29 (2008) 1989-2006), enzyme immobilization (Acta Biomater. 4 (2008) 1770-1777, Biomaterials 29 (2008) 1118-1126), and tissue engineering scaffolds (Tissue Eng. 12 (2006) 1197-1211, Adv. Drug Deliv. Rev. 61 (2009) 1033-1042).

Among the various potential applications, drug delivery is one of the most promising uses. From the first study on the application of electrospun nanofibers for the sustained release of tetracycline hydrochloride by Kenawy et al. (J. Control. Release 81 (2002) 57-64), electrospun nanofibers have been successfully used to achieve different drug release profiles (Adv. Polym. Sci. 246 (2012) 241-262, J. Mater. Sci. - Mater. M. 16 (2005) 933-946). Among them immediate release formulations are most popular, as electrospinning provides the opportunity to improve dissolution and bioavailability, especially for poorly-soluble drugs. During the process amorphous materials with improved water solubility of the APIs are prepared (Eur. J. Pharm. Sci., (2013) 49 (4) 595-602). Moreover, the film can disintegrate quickly upon contact with water, due to a very large surface area of nanofibers and water soluble excipients used for their production. Thanks to these features ODFs can become a beneficial oral dosage forms, characterized by immediate release, even for hardly-soluble drugs under physiological conditions.

One of the most important requirements to use electrospinning for oral strips production is the preparation of the final formulation in the form of solution. When using water-soluble polymers water can be applied as solvent in the electrospinning process, however, it is not sufficient to dissolve poorly soluble drugs and requires the addition of organic solvents. Different solvents from this group (ethanol, methanol, dichloromethane, chloroform, trifluoroethanol, tetrahydrofuran) can be applied in the process of electrospinning, but this can be considered a disadvantage for the oral formulation due to the safety issues (Eur. J. Pharm. Sci., (2013) 49 (4) 595-602). Moreover, satisfactory taste masking of dissolved API and stability of the biphasic liquid mixture still remain a challenge.

### Disclosure of the Invention

The present invention relates to the composition of the stable liquid mixture used for electrospinning process in order to prepare orodispersible films, which comprises hardly soluble therapeutic agents, for example tadalafil or sildenafil (PDE5 inhibitors) together with one or more suitable pharmaceutical excipients. In the preferred embodiment the amount of API is 0.1- 10.0% by weight of the total liquid mixture / solution.

In another preferred embodiment the claimed composition of solution apart from API, consists of the water soluble polymers, taste masking agents and optionally sweeteners, aromas, flavors, antioxidants and stabilizers, which are completely dissolved in the mixture of at least two different solvents.

In another preferred embodiment the proper selection of polymeric excipients, used together or without antioxidants, provides sufficient purity profile of chosen APIs and still keep fast dissolution profiles.

The invention provides also the proper selection and proportion between solvents for obtaining stable viscous clear or colloidal solutions with completely dissolved API and other suitable excipients, which can be electrospun into homogenous layer, characterized by favorable properties, including acceptable purity without residual solvents.

The present invention also relates to the method of preparation of stable solution, comprising completely dissolved API and excipients mentioned above, used for electrospinning process. The invention further provides methods for formulation stabilization, consisting of one or more of the following steps: pH control, addition of reducing agents, complexation and using of nitrogen atmosphere during electrospinning process.

The invention further provides the effective two-step taste masking method, which led to reduction of the unpleasant bitterness of the incorporated drug substance, consisting of complexation step with cyclodextrin followed by addition of sweeteners and flavors.

In another preferred embodiment the pharmaceutical composition is in the form of fast dissolving strips intended for oral administration, characterized by sufficient purity and fast dissolution profile.

The invention provides a pharmaceutical composition of ODFs, comprising at least one of the poorly water-soluble active ingredients, together with water soluble polymers and one or more suitable pharmaceutical excipients, which is obtained by electrospinning of the claimed solution.

### Detailed description of Invention

The objective of the present invention is to provide a novel pharmaceutical composition of orodispersible films, prepared by electrospinning method, comprising hardly-soluble active pharmaceutical agents with favorable properties, including faster disintegration and suitable purity profile. Authors of the present invention surprisingly found that the proper selection and proportion between polymers as well as that proper selection and proportion between solvents result in clear or colloidal solution of chosen excipients and hardly soluble APIs, which can be effectively electrospun into homogenous layer of immediate disintegration, desirable content uniformity and low content of residual solvents.

The present invention relates to the composition of the stable solution used for electrospinning process in order to prepare orodispersible films, which comprises hardly soluble therapeutic agents, as tadalafil or sildenafil, together with one or more suitable pharmaceutical excipients. In the preferred embodiment the amount of API is 0.1- 10.0% by weight of the total solution. The claimed composition consists of:
- 2-30% of water soluble long-chained polymers such as, but not limited to cellulose and its derivatives (preferably methylcellulose, hydroxyethyl cellulose, hydroxypropyl cellulose, hydroxypropyl methylcellulose), polyoxyethylene glycols and their derivatives and copolymers, polyacrylic acid, its derivatives and copolymers, methacrylic acid derivatives, xanthan gum, alginates, carrageenan or their mixtures,
- 0.1-10% of taste masking agents, as α-, β-, γ-cyclodextrins and their derivatives and mixtures thereof
- up to 0,5% of sweeteners (preferably thaumatin, sucralose, aspartame, sodium saccharin) and their mixtures
- Optionally up to 5% of aromas and flavors (preferably mint, orange, lemon, strawberry, raspberry, cherry, ginger) and their mixtures
- Optionally up to 0.5% of antioxidants (preferably butylated hydroxyanisole - BHA, butylated hydroxytoluene - BHT, ascorbyl palmitate)
- optionally up to 2% of the pharmaceutically accepted colorants, which can be used in drug products (preferably E100, E101, E102, E104, E110, E120, E122, E123, E124, E131, E132, E133, E141, E142,E160a-e, E162)
- 10-90% of solvents (preferably water, isopropanol, ethanol, DMF, DMAC) and their mixtures.

The main components of the above formulation are long-chained water soluble polymers. In the present invention the mixture of hydroxypropyl methylcellulose, polyoxyethylene glycol and polyoxyethylene and polyoxypropylene glycol copolymer (different grades of Pluronic) was used. This selection of polymeric components led to achieve the high productivity of electrospinning process and obtain adequate mechanical properties of the final ODFs (elasticity, homogeneity, appearance of the films, quality of nanofibers). It was observed that electrospinning of the solution based either on HPMC alone or the mixture of HPMC and PVP/PVA led to obtain brittle and fragile films, while using PVP or PVA alone or in the mixture with PEG resulted in the stickiness and sensitivity to touch of the final ODFs.

Other water soluble polymers were also used to prepare solution for electro spinning. The mixture of hydroxypropyl methylcellulose, polyethylene glycol and a polyethylene glycol, polyvinyl acetate and polyvinylcaprolactam-based graft copolymer (Soluplus) was successfully electrospun. Moreover, it was surprisingly found out that addition of polyoxyethylene glycol derivatives in the formulation, alone or with β -cyclodextrins can help in effective masking of the unpleasant taste of APIs.

In order to achieve stable solution, polymeric ingredients (HPMC, PEG, Soluplus/Pluronic) were used in the particular ratios (preferably 9:1:1, 9:1:0,5, 8:2:1, 8:2:0,5, 7:3:1, 7:3:0,5, 7:1,5:1, 6:4:1, and 6:4:0,5 respectively). These proportions between polymers led to obtain clear solution, characterized by an adequate viscosity and physical stability for at least 3 days (no precipitation, viscosity change, liquid phase separation). Moreover, described polymeric composition, used together with or without antioxidant agents, provided low impurity profile of ODFs (specifically N-oxide level control) in comparison with commonly used used PVA (Express Polym. Lett., 4 (2010) 763-772) and PVP (J. Mater. Sci. - Mater. M. 16 (2005) 933-946, Reactive and Functional Polymers 99, (2016) 65-72). The preferable amount of polymer mixture in the solution was between 2-30% of the total weight.

One of the most important features of a rapidly disintegrating oral dosage form is a matter of taste. Masking the unpleasant taste of the drug substance in the ODF can be difficult, especially when drug form, quickly dissolving in the oral cavity, has a direct contact with the taste buds on the tongue, and the drug substance is incorporated into the polymer matrix from the solution (molecular dispersion).The invention further provides the effective two-step taste masking method, which led to reduction of the unpleasant bitterness of the incorporated API. It consists of complexation of API with cyclodextrins (preferably α-, β- or γ-cyclodextrins) followed by addition of sweeteners and flavors. This method resulted in effective reduction of bitter taste of API, despite the fact that complexation reaction did not proceed in the aqueous environment but the aqueous-organic mixture. Concentration of α-, β- or γ-cyclodextrins or their mixture in the solution was in the range from 0.1 to 10%, preferably 0.2-5%, most preferably 0.5-3%.

In order to obtain an attractive taste of the final ODFs, the formulation also comprises at least one sweetener (preferably thaumatin, sucralose, aspartame, sodium saccharin) at concentration up to 0.5% of total weight of solution, preferably up to 0.2%, optionally at least one flavor (preferably mint, orange, lemon, strawberry, raspberry, cherry, ginger) at concentration up to 5%, as well as optionally at least one of the pharmaceutically accepted colorants and dyes, at concentrations up to 2% of total weight of solution.

The invention provides also the proper selection and proportion between solvents, which result in obtaining of a clear or colloidal solution of chosen excipients and hardly soluble APIs. In the present invention a mixture of organic solvents and water (or buffer) e.g. dimethylacetamide, ethylene glycol, propylene glycol, PEG 200, PEG 300 (Macrogol 300), PEG 1000, PEG 10000, dimethylformamide (DMF), ethanol, THF, acetonitrile, isopropanol and water, mixed in different ratios (preferably 1:1:1, 1:2:1, 1:1:2, 2:1:1, 2:2:1, 2:1:2, 1:2:2, respectively) allowed for preparation of stable viscous colloidal solutions with completely dissolved API, which have been electrospun into homogenous layer, characterized by desirable content uniformity and low content of residual solvents. The proper ratios between solvents provided sufficient stability of hardly soluble API in the dissolved form and helped to avoid precipitation of ingredients from the solution.

The present invention also relates to the method of preparation of stable solution, comprising completely dissolved API and excipients mentioned above, used for electrospinning process. Stability of the solution depended not only on the appropriate balance between ingredients and solvents, but also on the suitable order of the preparation steps.

Following APIs can be used as single component, or in combination. Examples of useful drugs include antiemetics, antinauseants, cardiovascular agents such as, antianginal drugs, antiarrhythmics, anticoagulants, antithrombotic drugs, coronary dilators, antihypertensive drugs, central nervous system stimulants, dopamine receptor agonists anti-anxiety agents cholinesterase inhibitors, anesthetics, anticonvulsants, hypnotics, antidepressants, antiparkinsonian agents, psychotherapeutic agents, anti-diabetic agents, anti-cholesterolemics, premature ejaculation management agents, drugs used to treat erectile dysfunction, parasympatholytics, parasympathomimetics, motion sickness management agents, antidiarrheal preparations, antidotes, anti-histamines, anti-inflammatory agents, anti-lipid agents, analgesics, anti-asthmatics, anti-stroke agents, anti-thyroid preparations, antitumor drugs, antiviral agents, acne medications, alkaloids, amino acid preparations, dry cough agents, anti-uricemic drugs, antiviral drugs, anabolic preparations, systemic and non-systemic anti-infective agents, antineoplastics, antirheumatic drugs, appetite stimulants, biological response modifiers, blood modifiers, bone metabolism regulators, contraceptives, decongestants, dietary supplements, , endometriosis management agents, enzymes, fertility agents, gastrointestinal agents, hormones, hypercalcemia and hypocalcemia management agents, immunomodulators, immunosuppressives, migraine preparations, muscle relaxants, obesity management agents, osteoporosis preparations, oxytocics, prostaglandins, respiratory agents, sedatives, smoking cessation aids such as bromocryptine and nicotine, sympatholytics, anti-tremor preparations, urinary tract medications, vasodilators, laxatives, antacids, antipyretics, appetite suppressants, expectorants, anti-ulcer agents, anti-inflammatory substances, cerebral dilators, peripheral vasodilators, psycho-tropics, stimulants, vasoconstrictors, migraine treatments, antibiotics, tranquilizers, anti-psychotics, anti-tumor drugs, neuromuscular drugs, hyper- and hypoglycemic agents, thyroid and anti-thyroid preparations, diuretics, aritispasmodics, uterine relaxants, anti-obesity drugs, erythropoietic drugs, cough suppressants, mucolytics, DNA and genetic modifying drugs, proteins ,peptides and combinations thereof.

Non-limiting examples of active pharmaceutical agents used according to the present invention are dapoxetine, donepezil hydrochloride; ondansetron; desloratadine; olanzapine; risperidone; rivastigmine tartrate; sildenafil; vardenafil; tadalafil;yohimbine galantamine; diclofenac potassium; buprenorphine; naloxone; alprazolam; clonazepam; diazepam; lorazepam; sumatriptan; eletriptan; rizatriptan; zolmitriptan; naratriptan; almotriptan; frovatriptan; cetirizine hydrochloride; loratadine; ambroxol hydrochloride; apomo hine; ascorbic acid; betamethasone; caffeine; dextromethorphan; glimepiride; hydrocortisone; ketotifen; loperamide; meclozine; melatonin; neramexane; piroxicam and combinations thereof.

In a preferred embodiment of the present invention, the active pharmaceutical ingredient may comprise one or more anti-emetics. Such anti-emetics include and may be selected from one or more of the group consisting of: ondansetron, granisetron, palonosetron, dronabinol, aprepitant, ramosetron, metopimazine, nabilone, tropisetron, metoclopramide, prochlorperazine, trimethobenzamide, dimenhydrinate, prochlorperazine and dolasetron.

In another preferred embodiment of the present invention, the API is PDE5 inhibitor, preferably tadalafil.

The films of the present invention may be mucoadhesive, whose advantage resides in their ability to bypass the gastrointestinal tract, and barriers in the gastrointestinal tract to drug absorption such as first pass metabolism and decomposition of the active ingredient in the stomach.

Alternatively the films of the present invention may be non-mucoadhesive which mimics the pharmacokinetic profile conventional immediate release solid oral dosage forms and are bioequivalent to and interchangeable with existing orally administered drug products. The films of the present invention must be formed into a sheet or film prior to drying. After the desired components are combined to form a multi-component matrix, including the polymer, water, and active or other components as desired, the combination is formed into a film, by electrospinning method.

The method according to the present invention consists of the following points:
1. Dissolving of API and β -CD in the mixture of DMF and isopropanol (complexation step),
2. Addition of polymers, sweeteners and optionally other needed excipients selected from aromas and flavors, antioxidants, colorants
3. Dilution with water,
4. Heating up to approx. 50°C with continuous stirring (up to 30 min), followed by cooling to the room temperature.
5. Electro spinning

It was experimentally proved that any change in the order of solvent addition or dissolving the individual components led to excipients / API precipitation from a solution or caused phase separation in the final mixture.

The invention further provides methods for formulation stabilization, consisting of one or more of the following steps:
- using of pH modifiers and buffers for pH control (protonation on N reduced formation)
- addition of polymeric excipient
- addition of reducing saccharides
- optionally addition of thioglycolic acid, cysteine, methionine, ascorbic acid or any other reducing agents
- complexation with cyclodextrins
- electrospinning process (optionally in nitrogen atmosphere).

In another preferred embodiment the claimed composition of solution results in the following composition of oral strip:
- up to 30% of API
- up to 90% of water soluble long-chained polymers such as, but not limited to cellulose and its derivatives (preferably methylcellulose, hydroxyethylcellulose, hydroxypropylcellulose, hydroxypropylmethylcellulose), polyoxyethylene glycol and its derivatives and copolimers, polyacrylic acid, metacrylic acid derivatives or their mixtures,
- up to 30% of taste masking agents, as α-, β-, γ-cyclodextrins and their derivatives and mixtures thereof,
- Optionally up to 10% of aromas and flavors (preferably mint, orange, lemon, strawberry, raspberry, cherry, ginger) and their mixtures,
- Optionally up to 10% of sweeteners (preferably thaumatin, sucralose, aspartame, sodium saccharin) and their mixtures,
- Optionally up to 1% of antioxidants (preferably buthylated hydroxyanisole - BHA, buthylated hydroxytoluene - BHT, ascorbyl palmitate).

In presented invention ODFs comprise at least one pharmaceutically active substance, deposited in and/or on at least one active layer of nanofibers of biologically compatible material. Preferably, ODFs consist of more than one layer, of which at least one is prepared by electrospinning method and can be combined with other layers produced by any other method. In another preferred embodiments ODFs consist of more than one layer, where each layer can have the same or different composition. Preferably external polymeric layers have particular composition (i.e. polymers and taste masking excipients), while internal layer(s) consist(s) of different ingredients and contain only API in the matrix ("sandwich model"). In another preferred embodiment ODFs are prepared by electrospinning method in the way providing that the film is free of residual solvents.

In another embodiment solutions or dispersions are electrospun on supportive material such as, but not limited to polypropylene textile, baking paper, siliconized paper, edible paper, aluminium foil. The carrier can be continuously removed during the electrospinning process, or afterwards, before cutting of the film into pieces (PP textile, baking paper, aluminium foil). Preferably, it can be an integral part of the final product (edible paper).

### Examples

### Example 1

| **Ingredient** | **Composition** | | |
|---|---|---|---|
| | **% in solution** | **% in ODF** | **mg/ODF 27x35mm** |
| Tadalafil | 1.0 | 8.91 | 2.5 |
| HPMC | 7.0 | 62.37 | 17.5 |
| PEG 100 000 | 1.5 | 13.37 | 3.75 |
| Pluronic F127 | 1.0 | 8.91 | 2.5 |
| Hydroxy-β -cyclodextrin | 0.5 | 4.46 | 1.25 |
| sucralose | 0.02 | 0.18 | 0.05 |
| mint aroma | 0.2 | 1.78 | 0.50 |
| BHA | 0.002 | 0.02 | 0.005 |
| DMF | 30 | - | - |
| Isopropanol | 30 | - | - |
| Water | 30 | - | - |

### Pluronic F127 - block copolymer of ethylene oxide and propylene oxide

### Solution preparation:

0.5 g of hydroxy-β-cyclodextrin was dissolved in the mixture of isopropanol and DMF (30 g + 30 g), then 1.0 g of tadalafil wad added and the solution was left for approximately 5 min with continuous stirring. After this time polymers were dispersed in the solution (7.0 g HPMC, 1.5 g PEG and 1.0 g Pluronic), and sucralose (0.02g), mint aroma (0.2 g) and BHA (0.002 g) were added. The whole mixture was left for next 6 hours with continuous mixing. Then dispersion was diluted with water (29.6 g) and heated up to 50°C if needed, to obtain clear solution. Prepared polymeric solution comprising 8.9% of tadalafil was electrospun into 2-layer film, collected on the polypropylene textile. After separation from the carrier, the film was cut into 27 x 35 mm strips, containing 2.5 mg of tadalafil per strip.
One layer weight 1.45-1.55 mg/cm²
Tadalafil dose: 0.265 mg /cm²
Total impurities: 0.78%

### Process parameters:

Equipment: laboratory scale Nanospider - needleless electrospinning
Electrode distance [mm] 145
Rotation/wire speed [mm] 15
Rewinding speed [mm/min] 5
EMW speed [mm /sec] 180
Air Input- flow [m3/h] 60.0
Air Output- flow [m3/h] 110.0
High Voltage setup
   HV CE Supply current [mA] 0.07
   HV CE Supply voltage [kV] -15.0
   HV CE Supply current [mA] 0.07
   HV CE Supply voltage [kV] 25.0

### Example 2

| **Ingredient** | **Composition** | | |
|---|---|---|---|
| | **% in solution** | **% in ODF** | **mg/ODF 28x30 mm** |
| Tadalafil | 3.0 | 21.86 | 5.00 |
| HPMC | 7.0 | 51.01 | 11.67 |
| PEG 100 000 | 2.0 | 14.58 | 3.33 |
| Pluronic F127 | 1.0 | 7.29 | 1.67 |
| Hydroxy-β -cyclodextrin | 0.5 | 3.64 | 0.83 |
| sucralose | 0.02 | 0.15 | 0.03 |
| mint aroma | 0.2 | 1.46 | 0.33 |
| BHA | 0.002 | 0.01 | 0.0002 |
| DMF | 36 | - | - |
| Isopropanol | 36 | - | - |
| Water | 18 | - | - |

0.5 g of hydroxy-β-cyclodextrin was dissolved in the mixture of isopropanol and DMF (36 g of each), then 3.0 g of tadalafil wad added and the solution was left for 1 hour with continuous stirring. After this time polymers were dispersed in the solution (7.0 g HPMC, 2.0 g PEG and 1.0 g Pluronic), and sucralose (0.02 g), mint aroma (0.2 g) and BHA (0.002 g) were added. The whole mixture was left for next 6 hours with continuous stirring. Then dispersion was diluted with water (18 g) and heated up to 50°C, if needed to obtain clear solution. Prepared polymeric solution comprising 21.9% of tadalafil was electrospun into two-layer film, deposited on the polypropylene textile. After separation from the material, the film was cut into 28 x 30 mm strips, containing 5 mg of tadalafil per strip.
One layer weight 1.35-1.45 mg/cm²
Tadalafil dose: 0.6 mg /cm²
Total impurities: 0.53%

### Process parameters:

Equipment: laboratory scale Nanospider - needleless electrospinning
Electrode distance [mm] 150
Rotation/wire speed [mm] 15
Rewinding speed [mm/min] 5 (2x)
EMW speed [mm /sec] 350
Air Input- flow [m3/h] 60
Air Output- flow [m3/h] 110
High Voltage setup:
   HV CE Supply voltage [kV] 40
   HV CE Supply voltage [kV] -10

### Example 3

| **Solution I** | **Composition** | |
|---|---|---|
| | **% in solution** | **% in ODF layer** |
| Tadalafil | 2.5 | 16.67 |
| HPMC | 7.0 | 46.67 |
| PEG 100 000 | 2.0 | 13.33 |
| Pluronic F127 | 1.0 | 6.67 |
| Hydroxy- β -cyclodextrin | 2.5 | 16.67 |
| DMF | 36.0 | - |
| Isopropanol | 36.0 | - |
| Water | 18.0 | - |

| **Solution II** | **Composition** | |
|---|---|---|
| | **% in solution** | **% in ODF layer** |
| HPMC | 7.0 | 67.95 |
| PEG 100 000 | 3.0 | 29.12 |
| Thaumatin | 0.005 | 0.01 |
| Sucralose | 0.1 | 0.49 |
| Citric acid | 0.2 | |
| Lime aroma | 0.25 | 2.43 |
| Isopropanol | 45 | - |
| Water | 45 | - |

Solution I was prepared by dissolving 1.0 g of hydroxy-β-cyclodextrin in the mixture of isopropanol and DMF (36.0 g of each), then 2.5 g of tadalafil wad added and the solution was left for 1 hour with continuous stirring. After this time polymers were dispersed in the solution (7.0 g HPMC, 2.0 g PEG and 1.0 g Pluronic). The whole mixture was left for next 6 hours with continuous mixing. Then dispersion was diluted with water (18.0 g) and heated up to 50°C, if needed to obtain clear solution. The second solution was prepared by dissolving 7.0 g of hypromellose and 3.0 g of PEG in the mixture of isopropanol and water (1:1). 0.005 g of thaumatin, 0.1 g of sucralose and 0.25 g of lime aroma, citric acid 0.2 g were added at the end and mixed until dissolved. Final orodispersible film was achieved by electrospinning of both solutions, where the first layer was prepared from solution II and the second and the third layer were obtained from solution I. After separation from the supportive material, three-layer film was subsequently cut into 20 x 30 mm strips, containing 5 mg of tadalafil per strip.
Whole film weight 5.6 - 5.8 mg/cm²
Tadalafil dose: 0.83 mg / cm²
Total impurities: 0.19%

### Process parameters:

Equipment: Nanospider - needleless electrospinning
The first layer: Electrode distance [mm] 150
   Rotation/wire speed [mm] 15
   Rewinding speed [mm/min] 5
   EMW speed [mm /sec] 400
   Air Input- flow [m3/h] 60.0
   Air Output- flow [m3/h] 110.0
   High Voltage setup
   HV CE Supply voltage [kV] -10.0
   HV CE Supply voltage [kV] 35.0
Second and third layer:
   Electrode distance [mm] 150
   Rotation/wire speed [mm] 15
   Rewinding speed [mm/min] 5
   EMW speed [mm /sec] 350
   Air Input- flow [m3/h] 60.0
   Air Output- flow [m3/h] 110.0
   High Voltage setup
   HV CE Supply voltage [kV] -10.0
   HV CE Supply voltage [kV] 20.0

### Example 4

| **Ingredient** | **Composition** | | |
|---|---|---|---|
| | **% in solution** | **% in ODF** | **mg/ODF 20x19 mm** |
| Tadalafil | 2.5 | 16.3 | 5.00 |
| HPMC | 7.0 | 45.7 | 14.1 |
| PEG 100 000 | 2.0 | 13.1 | 4.05 |
| Pluronic F127 | 1.0 | 6.5 | 2.00 |
| Hydroxy-β -cyclodextrin | 2.5 | 16.3 | 5.00 |
| thaumatin | 0.005 | 0.03 | 0.01 |
| sucralose | 0.05 | 0.3 | 0.1 |
| mint aroma | 0.25 | 1.6 | 0.5 |
| DMF | 36 | - | - |
| Isopropanol | 36 | - | - |
| Water | 18 | - | - |

2.5 g of hydroxy-β-cyclodextrin was dissolved in the mixture of isopropanol and DMF (36 g of each), then 2.5 g of tadalafil wad added and the solution was left for 1 hour with continuous stirring. After this time polymers were dispersed in the solution (7.0 g HPMC, 2.0 g PEG and 1.0 g Pluronic), and sucralose (0.05 g), mint aroma (0.25 g) and thaumatin (0.005 g) were added. The whole mixture was left for next 6 hours with continuous mixing. Then dispersion was diluted with water (18 g) and heated up to 50°C, if needed to obtain clear solution. Prepared polymeric solution comprising 21.9% of tadalafil was electrospun into two-layer film, collected on the polypropylene textile. After separation from the carrier, the film was cut into 19 x 20 mm strips, containing 5 mg of tadalafil per strip.
One layer weight 4.0-4.2 mg/cm²
Tadalafil dose: 0.65 mg /cm²
Total impurities: 0.09%

### Process parameters:

Equipment: Nanospider - needleless electrospinning
Electrode distance [mm] 150
Rotation/wire speed [mm] 15
Rewinding speed [mm/min] 5
EMW speed [mm /sec] 350
Air Input- flow [m3/h] 60.0
Air Output- flow [m3/h] 110.0
High Voltage setup
HV CE Supply voltage [kV] -10.0
HV CE Supply voltage [kV] 40.0

## Claims

1. A method of manufacturing an orodispersible film composition of PDE5 inhibitor, which is tadalafil, **characterized in that** the method comprises preparing a liquid mixture of PDE5 inhibitor and at least one pharmaceutical acceptable excipient and electro spinning the liquid mixture to obtain the orodispersible film, whereas the method includes the following 5 steps:
a) dissolving API and β-cyclodextrine in an organic solvent selected from isopropanol, ethanol, dimethylformamide, N,N-dimethylacetamide and their mixtures, preferably the mixture of solvents DMF and isopropanol;
b) addition of polymers, taste masking agents, sweeteners and optionally aromas and flavors, antioxidants and colorants;
c) dilution with water;
d) heating up to approx. 50°C with continuous stirring (up to 30 min), followed by cooling to the room temperature to obtain liquid mixture; and
e) electrospinning the liquid mixture to obtain the orodispersible film.

2. A method according to claim 1, **characterized in that** liquid mixture is clear or colloidal solution.

3. A method according to claim 1 or 2, **characterized in that** the liquid mixture comprises PDE5 inhibitor and one or more excipients selected from water soluble polymers, solvents, taste masking agents, sweeteners and optionally aromas and flavors, antioxidants and colorants.

4. A method according to claim 3, **characterized in that** the amount of PDE5 inhibitor in the solution is 0.1- 10.0% w/w, amount of water soluble polymers is 2-30% w/w, amount of solvents is 10-90% w/w, amount of taste masking agents is 0.1-10% w/w, amount of sweeteners is 0.1-0.5% w/w, amount of aromas or flavors is up to 5% w/w, amount of antioxidants is up to 0.5% w/w, amount of colorants is up to 2% w/w by weight of the total liquid mixture.

5. A method according to claim 3-4, **characterized in that** the water soluble polymer is selected from cellulose and its derivatives, preferably methylcellulose, hydroxyethyl cellulose, hydroxypropyl cellulose and hydroxypropyl methylcellulose, or polyoxyethylene glycols and their derivatives and copolymers, or polyacrylic acid, its derivatives and copolymers, or methacrylic acid derivatives, or xanthan gum, or alginates, or carrageenan or their mixtures.

6. A method according to claim 5, **characterized in that** the water soluble polymer is preferably combination of hydroxypropyl methylcellulose, polyethylene glycol and a copolymer of ethylene oxide and propylene oxide or combination of hydroxypropyl methylcellulose, polyethylene glycol and a graft copolymer polyethylene glycol - polyvinyl acetate - polyvinylcaprolactam.

7. A method according to claim 6, **characterized in that** individual polymers of the polymer combination are used in the ratios selected from 9:1:1, 9:1:0.5, 8:2:1, 8:2:0.5, 7:3:1, 7:3:0.5, 7:1.5:1, 6:4:1, and 6:4:0.5.

8. A method according to claim 3-4, **characterized in that** the solvent is selected from water, isopropanol, ethanol, dimethylformamide, N,N-dimethylacetamide and their mixtures.

9. A method according to claim 8, **characterized in that** the solvent is preferably combination of dimethylformamide, isopropanol and water in ratios 1:1:1, 1:2:1, 1:1:2, 2:1:1, 2:2:1, 2:1:2, or 1:2:2.

10. A method according to claim 3-4, **characterized in that** the taste masking agent is selected from α-, β-, γ-cyclodextrins and their derivatives and their mixtures.

11. A method according to claim 3-4, **characterized in that** the sweetener is selected from thaumatin, sucralose, aspartame, sodium saccharin and their mixtures.

12. A method according to claim 3-4, **characterized in that** the aroma or flavor is selected from mint, orange, lemon, strawberry, raspberry, cherry, ginger and their mixtures.

13. A method according to claim 3-4, **characterized in that** the antioxidant is selected from butylated hydroxyanisole - BHA, butylated hydroxytoluene - BHT, ascorbyl palmitate.

14. An orodispersible film composition of PDE5 inhibitor, comprising:
5- 30% of PDE5 inhibitor, preferably tadalafil;
10- 90% of a water soluble polymer selected from cellulose and its derivatives, preferably methylcellulose, hydroxyethyl cellulose, hydroxypropyl cellulose and hydroxypropyl methylcellulose, or polyoxyethylene glycols and their derivatives and copolymers, or polyacrylic acid, its derivatives and copolymers, or methacrylic acid derivatives, or xanthan gum, or alginates, or carrageenan or their mixtures;
1- 30% of a taste masking agent selected from α-, β-, γ-cyclodextrins and their derivatives and mixtures thereof;
0.5- 10% of sweeteners selected from thaumatin, sucralose, aspartame, sodium saccharin and their mixtures;
optionally up to 10% of aromas and flavors selected from mint, orange, lemon, strawberry, raspberry, cherry, ginger and their mixtures;
optionally up to 1% of antioxidants selected from butylated hydroxyanisole - BHA, butylated hydroxytoluene - BHT, ascorbyl palmitate; and
optionally up to 2% of colorants.

15. An orodispersible film composition according to claim 14, comprising:
8-22% of Tadalafil;
60-85% of a water soluble polymer, which is combination of hydroxypropyl methylcellulose, polyethylene glycol and a copolymer of ethylene oxide and propylene oxide or combination of hydroxypropyl methylcellulose, polyethylene glycol and a graft copolymer polyethylene glycol - polyvinyl acetate - polyvinylcaprolactam;
5-20% of a taste masking agent, which β-cyclodextrin;
0.1-2% of a sweetener, which is sucralose and/or thaumatin;
1-2% of an aroma, which is mint and/or lime aroma;
0.0-0.02% of an antioxidant, which is butylated hydroxyanisole - BHA.
